# EUROPEAN PATENT SPECIFICATION

(11) **EP 3 901 338 B1**
(45) Date of publication and mention of the grant of the patent: **01.05.2024**
(21) Application number: 21169302.3
(22) Date of filing: 20.04.2021
(51) Int. Cl.: A41D 13/11, D03D 1/00

(54) **RESPIRATORY MASK AND METHOD OF PRODUCTION**
ATEMMASKE UND VERFAHREN ZUR HERSTELLUNG
MASQUE RESPIRATOIRE ET PROCÉDÉ DE PRODUCTION

(30) Priority: 20.04.2020 EP 20170451
(43) Date of publication of application: 27.10.2021
(73) Proprietor: Sanko Tekstil Isletmeleri San. Ve Tic. A.S., 16400 Inegol - Bursa (TR)
(72) Inventor: KONUKOGLU, Fatih, 16400 Inegol - BURSA (TR); LOYAN, Kenan, 16400 Inegol - BURSA (TR); TUNCER, Esref, 16400 Inegol - BURSA (TR); ÖZKAN, Ahmet, 16400 Inegol - BURSA (TR); ÖZDEMIR, Mahmut, 16400 Inegol - BURSA (TR); ÖZDEN, Erdogan Baris, 16400 Inegol - BURSA (TR); KILIÇKAN, Esin Kalfa, 16400 Inegol - BURSA (TR); ÖMER, Yilmaz, 16400 Inegol-Bursa (TR)
(74) Representative: Gislon, Gabriele

(56) References cited:
- WO-A1-97/10026
- WO-A1-2012/030798
- CN-A- 109 463 819
- DE-U1-202020 101 910

## Description

### Field of the invention

The present invention relates to respiratory masks and to a method of their production. More specifically, the present invention relates to a protection mask to be worn on the face. The invention also relates to a method for the production of this mask.

### State of the art

Personal respiratory masks, also known as "face masks" or "filtering face masks", are protective devices used to protect the wearer's respiratory system from airborne particles. Facial masks are in fact worn over the nose and mouth of the user to protect him from unwanted material suspended in the air. In some embodiments (namely those without a valve that let breath exit the mask) the mask also acts as a filter to prevent or reduce the leakage of any particles suspended in the user's breath to protect other people from possible infections of the person wearing mask. Known masks are typically made of non-woven fabric in two forms: a cup-shaped shape or a flat shape in which the non-woven fabric is partially folded on itself to be able to adapt to the shape of the face when worn. One type of flat mask is known as a "surgical mask". Flat masks made of woven fabric are also known.

CN109463819A, which discloses the preamble of claim 1, discloses a seamless knitted leak-proof mask. The mask is integrally formed by knitting technology. The mask consists of a middle part, an edge part and a strap. The middle part supports the shape of the high elastic part of the edge. The high elastic edge part of the mask can be deformed with the movement of the face, so that the edge part of the mask can always keep fit with the human face under any circumstances and the airtightness is improved.

WO 97/10026A1 relates to a mask including a cover material designed to cover a portion of a wearer's face. The mask further includes a hypoallergenic, anisotropic elastic material which stretches substantially in only one direction. The mask secured by anisotropic elastics is excellent at filtering contaminants.

A mask requires the presence of straps or bands, preferably of elastic material, which generally are in the form of loops that pass around the user's ears or around the user's head to keep the mask in the desired position on the user's face. The straps or equivalent retaining means are typically made separately and are attached to the body of the mask, by means such as sewing, gluing, ultrasonic welding, stapling or other means commonly known to those skilled in the art. Protective devices are also known in which the retaining means are loops of elastic material attached to a folded portion of the mask body.

The production of flat masks of the so-called surgical type discussed above is normally carried out with automatic machines that carry out in line all or almost all the necessary operations, such as cutting and folding the portion of non-woven fabric, welding or otherwise constraining the elastic straps that hold the body of the mask on the user's face and attaching the metal strip to the top side of the mask.

A face mask also requires the presence of a strip of plastically bendable material, generally a metal strip, that is located at the upper edge of the mask, i.e. at the side of the mask that is transversally floating over the bridge of the nose of the user when the mask is worn. This so-called "nose clip" may actually be made of any material provided it can be easily bended in a shape fitting to the bridge of the nose of the user to improve the air tightness of the mask.

One problem with known non-woven masks is that they can normally only be used only once and that they are difficult to be sanitized. This problem results in an increase in costs each time the mask is replaced, and in an increase in the mass of special waste to be treated.

Woven fabric masks are known that can be washed or otherwise sanitized to be used several times. However, woven fabric masks are more expensive than the non-woven fabric masks.

### Summary of the invention

An aim of the present invention is to solve the above discussed problems. More specifically, an object of the present invention is to provide a face protection mask which is made of woven fabric and that is at the same time inexpensive to manufacture. Another aim of the invention is to provide an inexpensive method of production of a protection mask in woven fabric.

These and other aims are reached by the respiratory mask of claim 1 and by the method of claim 8 for producing said mask of the present invention as described below.

In greater detail, the invention relates to a respiratory mask including a mask body, a strip of plastically bendable material and holding straps for holding the mask body to the face of the user, the mask body being made of a woven fabric having weft yarns and warp yarns, characterized in that at least one of said strip of plastically bendable material and holding straps comprises at least one yarn woven with said mask body fabric.

In an embodiment, the strip of bendable material is made of at least one, preferably a plurality of, metallic wire, a metallic filament yarn or metallic staple yarn that are woven into the body of the fabric in the weaving step of the mask production method. Suitable metallic yarns are copper filaments or steel filaments e.g. having diameter of 0.06 mm; in an embodiment of the yarn the metal wire is coated with a resin, e.g. an epoxy resin, to prevent metal oxidation and metal conductivity. The number of yarns or wires can be between 1 to 25, preferably 1 to 10. The fabric weave can be any one of plain, twill or sateen. The metallic wires extend in the fabric along the entire side of the mask from one edge of the mask body to the other edge.

In an embodiment, the holding straps are made of at least one elastic thread woven into the body of the fabric during the weaving step. Suitable elastic threads include braiding yarns, woven yarns, knitting yarns or a single elastic yarn. A suitable elastic thread has a composition of 85% elastane and 15% nylon, a diameter of 3 mm and a linear mass of 0.8 grams/meter.

According to an embodiment, the elastic thread is tightly woven, e.g. with a 1/1 or 2/1 pattern at the region of the fabric that is close to the mask sides; this weaving pattern secures the thread to the fabric. To provide for the strap or band required, the elastic thread has at least one long float over warp (or weft) yarns; several long floats may be provided, with holding point made e.g. by one or two weft or warp yarns that float over the elastic thread to hold it to the fabric portion. In an embodiment the elastic thread floats over about e.g. 3 - 5 cm of fabric before being held down by said weft or warp yarns; another float is provided with another holding point and so on along the fabric. Before using the mask, these holding yarns can be broken to detach the central part of the elastic thread from the fabric body and provide a loop of the required length to be passed over the ears or over the head of the user.

Preferably the metallic yarns and the elastic threads are both woven in the fabric. In an embodiment, metallic and elastic yarns are woven into the fabric as warp yarns. They may be both weft yarns or both warp yarns; in an embodiment, at least the elastic thread is woven as a warp yarn. Generally, if the elastic thread is to be passed over the ears, it is woven perpendicular to the metallic yarns; if the elastic thread is to be passed over the head, it is woven alongside the metallic yarns.

According to other embodiments, the sides of the mask may be provided with fusion yarns, i.e. yarns that have the ability to become softened, flattened or compressed and to hold together the fabric yarns. Suitable fusion yarns can soften and be flattened or compressed under heat, pressure or by a chemical treatment. In addition, gluing and waxing can be applied to reduce hairiness at the sides of the mask fabric. Suitable fusion yarns are low melting nylon commercially available, e.g. from Coats under the code Fusion-FTL2180. These yarns have a melting temperature of 85°C; upon treatment with e.g. hot steam the polymer softens and melts to incorporate in the polymer the loose ends of the yarns and prevent fabric fraying.

The body of the mask is made of a woven fabric. The fabric yarns can include man made or natural fiber or a blend of them. The fabric may include elastic yarns in warp or weft or both directions; preferably the weft yarns are elastic yarns and the warp yarns are non-elastic yarns, e.g. cotton yarns or polyester yarns. In a preferred embodiment the fabric includes weft yarns having different elasticity in different regions of the mask body, so as to enable the face mask to adapt and fit to the wearer's face. The weft yarns are vowen according to a fabric construction to improve fitting performance of the woven fabric. Exemplary suitable fabric weaves can be any one of plain, twill or sateen.

In embodiments, the cotton inelastic warp yarns have a count of 30 to 45 Ne, the elastic weft yarns have a count of 25 to 40 Ne. A typical fabric composition is 98% cotton and 1.68% elastan, the remaining part including metal yarns and fusion yarns, if present.

The invention woven face mask is manufactured by known weaving machines, preferably jacquard and dobby machines. An object of the present invention is a mask production method wherein a mask fabric is woven from warp and weft yarns, characterized in that said warp or weft yarns include at least one of: a yarn of plastically bendable (malleable) material and a yarn including an elastic thread that are woven with the fabric yarns to provide at least one of: a strip of plastically bendable material for a nose clip and holding straps for holding the mask body to the face of the user.

In an embodiment, the method includes the steps of providing a face mask design; providing a plurality of fabric yarns to produce a woven fabric; providing a plurality of metallic yarn, elastic threads to be introduced into the weaving machine and woven with said fabric yarns in a single production process.

The fabric as obtained on the loom includes a plurality of facemasks that are adjacent to each other in the fabric; they are thus produced together in an amount that depends on the width of the loom in the weaving machine and on the dimensions of the mask. After the required finishing steps the fabric is cut into individual masks, each mask being already provided with at least a "nose clip" or with the elastic straps, or preferably with both features.

According to an embodiment, fusion yarns are woven at the edges of the mask body with the fabric yarns and the technical yarns (i.e. metallic yarns and/or elastic thread) to provide a finishing of the mask by heat treatment of the fabric. Thus, the invention method may include a heat treatment step such as e.g. hot steam treatment of the fabric.

The fabric is then cut to provide single masks that advantageously are provided with all required items, including elastic straps and a malleable (plastically bendable) material for a nose clip. Subsequent finishing steps such as trimming, folding, or gluing may be used to give the final form of the mask.

### Brief description of the drawings.

The invention will now be disclosed with reference to the following schematic and exemplary, non-limiting drawings in which:
Fig. 1 is a schematic view of a mask according to the invention;
Fig. 2 is a detailed view of the mask according to fig. 1 showing enlarged details of possible weaving patterns;
Fig. 3 is a schematic view of a section warpwise of the mask of the invention;
Fig. 4 is a schematic view of the back side of a mask of the invention and
Fig. 5 is a schematic view of the mask in use.

### Disclosure of preferred embodiments.

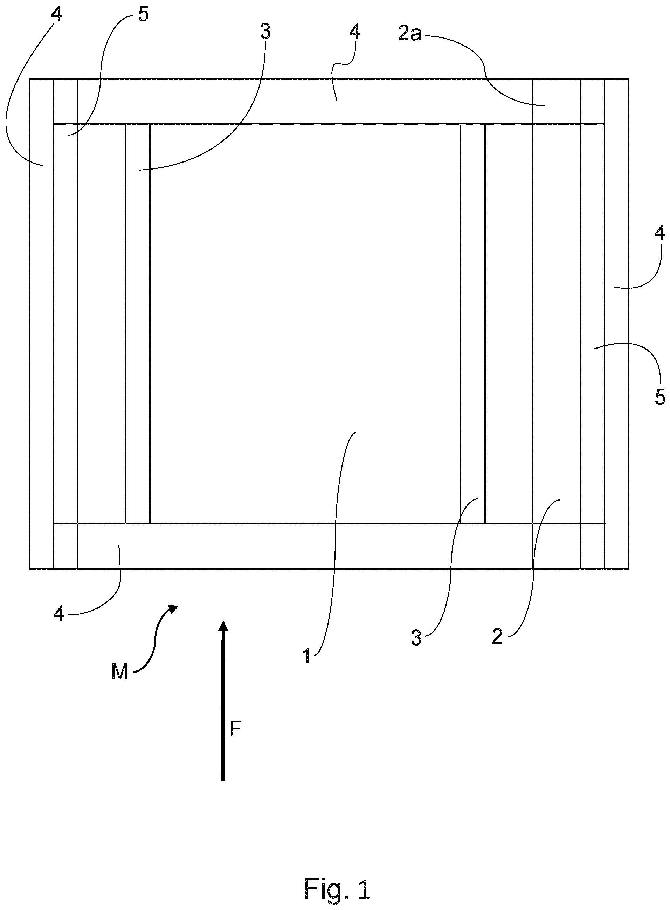

With reference to fig. 1, the mask M includes a mask body made of a woven fabric 1 including warp yarns and weft yarns; in fig. 1 the warp yarns extend vertically, i.e. parallel to arrow F that shows the machine direction or longitudinal direction of the fabric. Mask M is obtainable from a woven fabric comprising a plurality of adjacent masks as above discussed.

Woven with the fabric yarns are also present further yarns that provide at least one of a strip 2 of plastically bendable material forming a so-called "nose clip" and holding straps 3, preferably elastic holding straps that pass around the head of the user when the mask is used. According to the invention, the yarns that form strip 2 and strap 3 are woven with the other fabric yarns into the fabric of the mask during the weaving step. Said yarns define respective portions, or regions, of the mask.

The yarns that form strip 2 of bendable material for the nose clip may comprise a plurality of metallic wires, such as a metallic filament yarn or metallic staple yarn that are woven into the body of the fabric in the weaving step of the mask production method. Suitable metallic yarns are copper filaments or steel filaments having diameter of 0.06 mm, to prevent metal oxidation the metal wire is preferably coated with a resin, such as an epoxy resin. A suitable diameter is 0,05-0,10 mm, preferably 0,06 mm incuding coating layer. The number of metal yarns or wires can be between 1 to 25, preferably 1 to 10. As visible in the figures, the strip 2 of metallic wires extend in the fabric along the entire side of the mask from one edge 8 of the mask body to the other edge 9; similarly, the elastic thread will extend through the entire body of the mask.

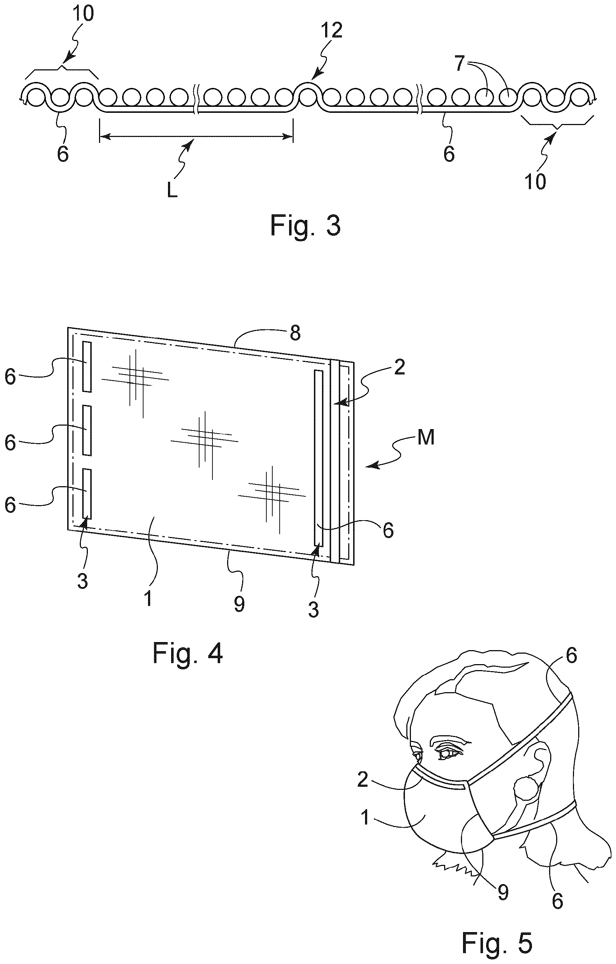

Holding straps 3 are made of at least one elastic thread 6 (see fig. 3) woven into the body of the fabric during the weaving step. In an embodiment, thread 6 is a single elastic yarn having a composition of 85% elastane and 15% nylon, a diameter of 3 mm and a linear mass of 0.8 grams/meter.

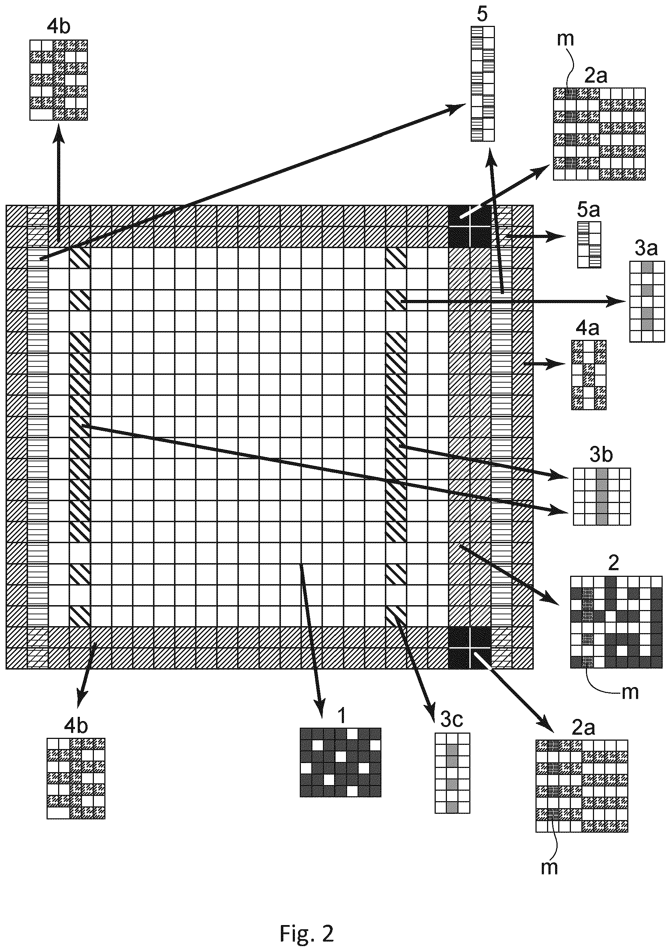

The weaving pattern of thread 6 is such that at the ends 10 and 11 (fig. 3) of elastic thread 6, i.e. at the area of the fabric that is close to sides 8 and 9 of the mask, the elastic thread is tightly woven, e.g. with a 1/1 or 2/1 pattern. To provide for the strap or band required, the elastic thread has at least one long float under warp (or weft) yarns. The embodiment shown in figure 2 is provided with one long float; in fig. 3 a thread 3 with several long floats is shown. In this embodiment thread 3 is kept adherent to the body of fabric 1 by means of several holding points 12 made by one weft yarn that floats over the elastic thread to hold it to the fabric portion at said holding point. The length L of thread 6 under portions, i.e. the length L of the portion of yarn 6 between two adjacent holding points 12, may be about e.g. 3-5 cm of fabric. As previously discussed, before using the mask, weft yarns at holding points 12 are broken to detach the elastic yarn from the fabric body and to provide a loop of the required length to be passed over the head of the user, see fig. 5.

In the shown embodiment, elastic yarns 6 are woven into the fabric as warp yarns to provide an elastic strap or band to be passed over the head; in this embodiment, one of the elastic yarns 6 is woven parallel to the metallic yarns of strip 2. The face of the mask where the under portions of the elastic thread are located is the side of the mask that will be in contact with the face of the user (see fig. 5).

Advantageously, fusion yarns are provided along the sides of the mask in the peripheral, i.e. in the contour portion 4 of the mask (see Figures 1 and 2); fusion yarns are yarns that have the ability of becoming softened and to be flattened or compressed to hold together the fabric yarns at the edge of the fabric to prevent loose ends being visible. Suitable fusion yarns can be softened and flattened or compressed under heat, pressure or by a chemical treatment. Suitable fusion yarns are low melting nylon commercially available, e.g. from Coats under the code Fusion- FTL2 180. These yarns have a melting temperature of 85°C.

The mask construction also preferably includes two warp-wise portions, or regions 5, of so called limiting yarns, e.g. polyester yarns that define the body of the mask between the mask body yarns or the metallic or elastic yarns and the peripherally located fusion yarns of mask region 4.

The body 1 of the mask is made of a woven fabric from yarns that may include man made or natural fibers or a blend of them. The fabric may include elastic yarns in warp or weft or both directions; according to one embodiment the weft yarns are elastic yarns and the warp yarns are non-elastic yarns, e.g. cotton yarns or polyester yarns. Preferably the weft yarns have different elasticity in different regions of the mask body; namely, the weft yarns closer to sides 8 and 9 have less elongation than the yarns in the central area of the mask, so as to enable the face mask to adapt and fit to the wearer's face. Suitable fabric weaves for the mask body 1 can be any one of plain, twill or sateen.

Typically, the cotton inelastic warp yarns have a count of 30 to 45 Ne, the elastic weft yarns have a count of 25 to 40 Ne. A typical fabric composition is 98% cotton and 1.68% elastan, the remaining part including metal yarns and fusion yarns, if present. The count and the warp and weft densities (after washing) are selected to provide the fabric 1 with the required filtering properties; said filtering properties may be assessed according to standard EN 14683:2019.

The schematic and merely exemplary weave report of fig. 2 shows the above mentioned regions of the mask fabric; for each region of the fabric an enlarged sample of construction is shown, to provide details of a possible embodiment of the invention. In a mask of the present invention, each of the portions shown in Figure 1 may be woven according to patterns different from the ones shown in fig. 2; in other words, the exemplified patterns do not necessarily have to be present all together as shown in fig. 2.

In the following exemplary description, the mask body portion 1 is woven by cotton weft yarn and cotton warp yarn. The weft yarn is shown in fig. 2 with white whereas the warp yarn is shown with black in the weaving pattern.

Portion 4 of the mask comprises fusion yarns. In an embodiment, the part of portion 4 that extends in warp direction, i.e. parallel to arrow F, is referred to with numeral reference 4a and has a weaving scheme where the weft yarn is a cotton yarn (shown with white) and the warp yarn is a fusion yarn (shown with a grey pattern). In the part of region 4 extending in the weft direction (shown with reference 4b), the fusion yarns are weft yarns shown in white and the warp yarns shown in grey pattern are cotton yarns. Region 3 shown in Figure 2 is woven by a cotton weft yarn shown with white in the pattern and an elastic warp yarn shown with grey. The elastic yarn is located on the back side of the fabric as shown in fig. 3 and fig. 4. Examples of weaving reports of top part, middle part and bottom part of region 3 are shown in figures 3a, 3b and 3c, respectively.

In mask portion 5, the weft yarn is a cotton yarn shown with white in the weaving pattern whereas the warp yarns are "limiting" yarns (e.g. in polyester) and are shown with grey pattern. In portion 5a where regions 4 and 5 overlap, the weft yarns are fusion yarns shown in white and the warp yarns are polyester yarns as shown in region 5.

Mask portions 2 and 2a comprise at least one metallic warp yarn (referred to with reference m), white weft yarns and black warp yarns. The overlapping area of regions 2 and 4 is identified with reference number 2a. For example portion 2a shows a possible weave of the overlapping regions 2 and 4. In detail, the weaving pattern of portion 2 includes a cotton weft yarn (in white) and two different warp yarns formed by a cotton yarn (in black) and a metallic warp yarn (m). The weaving pattern of region 2a is formed by a fusion weft yarn (white) and by two different warp yarns which are a cotton yarn (in grey) and the metallic yarn (m).

The width of each region is designed according to the specific requirement of the mask, which means that the number of the weaving patterns shown in Figure 2 may be chosen to provide the best advantageous effects of the present invention. For example, weftwise fusion region 4b may include seven weft yarns as shown in 4b or might include more or less weft yarns.

The method of producing the above discussed mask provides weaving warp and weft yarns, said warp or weft yarns including at least one of a yarn of plastically bendable yarns and at least one yarn including an elastic thread 6. Said yarns are woven with the fabric yarns to provide at least one of a strip 2 of plastically bendable (malleable) material and a pair of holding straps 3 for holding the mask body to the face of the user.

The method may also include the steps of providing a facemask design; providing a plurality of fabric yarns to produce a woven fabric; providing a plurality of metallic yarn, elastic threads to be introduced into the weaving machine and woven with said fabric yarns in a single production process.

The fabric on the loom is made to include a plurality of face masks that are all produced together depending on the width of the loom in the weaving machine and on the dimensions of the mask.

Fusion yarns are woven with the fabric yarns and the technical yarns (metallic yarns and/or elastic thread) to provide peripheral region 4 along the sides of the mask fabric 1. The fabric is subjected to a finishing step by a heat treatment step such as e.g. hot steam treatment of the fabric. In this step the low melting fusion yarns soften or melt to a sufficient degree to hold together the ends of the yarns and avoid fabric fraying. Surface treatment of the fabric may be carried out.

The fabric is then cut to provide single masks that comprise all required items, including elastic straps and a malleable (plastically bendable) material for a nose clip. Subsequent finishing steps such as trimming, folding, or gluing may be used to give the final form of the mask.

## Claims

1. A respiratory mask including a mask fabric body (1), a strip of plastically bendable material (2) and holding straps (3, 6) for holding the mask body to the face of a user, the mask body being made of a woven fabric **characterized in that**: said woven fabric has weft yarns and warp yarns, said strip of plastically bendable material comprises at least one yarn woven with said mask body fabric and/or said holding straps comprise at least one yarn woven with said mask body fabric.

2. A mask according to claim 1, wherein said strip of bendable material is made of at least one, preferably a plurality of metallic filament yarns (m) preferably coated with a resin.

3. A mask according to claim 1 or 2, wherein said holding straps (3) are made of at least one elastic thread (6) woven into the body of the fabric during the weaving step.

4. A mask according to claim 3, wherein said elastic thread is tightly woven at the region of the fabric that is close to the mask sides (8, 9), said thread (6) having at least one long float over warp (or weft) yarns, preferably several long floats in a central region of the mask fabric body (1).

5. A mask according to claim 4, wherein said central portion of the mask further comprises holding points (12) made by at least one weft or warp yarn floating over said elastic thread (6) to hold it to the fabric portion (1).

6. A mask according to any previous claim, wherein said metallic and elastic yarns are woven into the fabric as warp yarns.

7. A mask according to any previous claim, wherein the peripheral sides (4) of the mask are provided with fusion yarns.

8. A method of producing a mask according to any previous claim, comprising the step of providing a plurality of fabric yarns to produce a woven fabric; **characterized in** comprising the step of weaving warp and weft yarns, said warp or weft yarns including at least one of a plastically bendable yarn and at least one yarn including an elastic thread (6) to provide at least one of a strip (2) of plastically bendable material for a nose clip and a holding strap (3) for holding the mask body to the face of the user.

9. The method of claim 8 wherein the fabric on the loom includes a plurality of facemasks that are eventually cut after the fabric is removed from the loom and has gone through finishing steps.

10. The method of claim 8 or 9, wherein fusion yarns are woven with the fabric yarns to provide fusion regions (4) along the sides of the mask fabric (1).

11. The method of claim 10, further including a heat treatment step whereby low melting fusion yarns soften or melt to a sufficient degree to hold together the ends of the yarns.

12. The method of any claim 8 to 11, further comprising finishing steps including at least one of surface treatment, trimming, folding, or gluing.

13. The method of any claim 8 to 12, wherein said mask further comprises several long floats of said elastic threads on one side of the mask and several holding points (12) made by at least one weft or warp yarn floating over said elastic thread (6) to hold it to the fabric portion (1), the method including the step of breaking said holding yarns (12) to provide a long loop of the elastic thread (6).

## Patentansprüche

1. Atemmaske mit einem Maskengewebekörper (1), einem Streifen aus plastisch biegbarem Material (2) und Haltebändern (3, 6) zum Halten des Maskenkörpers am Gesicht eines Benutzers, wobei der Maskenkörper aus einem gewebten Stoff besteht, **dadurch gekennzeichnet, dass** der gewebte Stoff Schussgarne und Kettgarne aufweist, wobei der Streifen aus plastisch biegbarem Material mindestens ein mit dem Maskenkörpergewebe verwobenes Garn aufweist und/oder die Haltebänder mindestens ein mit dem Maskenkörpergewebe verwobenes Garn aufweisen.

2. Maske nach Anspruch 1, wobei der Streifen aus biegbarem Material aus mindestens einem, vorzugsweise aus einer Vielzahl von metallischen Filamentgarnen (m) hergestellt ist, die vorzugsweise mit einem Harz beschichtet sind.

3. Maske nach Anspruch 1 oder 2, wobei die Haltebänder (3) aus mindestens einem elastischen Faden (6) hergestellt sind, der während des Webschritts in den Gewerbekörper eingewebt ist.

4. Maske nach Anspruch 3, wobei der elastische Faden in dem Bereich des Gewebes, der nahe an den Maskenseiten (8, 9) liegt, eng gewebt ist, wobei der Faden (6) mindestens eine lange Flottierung über dem Ketten- (oder dem Schuss-) garn aufweist, vorzugsweise mehrere lange Flottierungen in einem zentralen Abschnitt des Maskengebekörpers (1).

5. Maske nach Anspruch 4, wobei der zentrale Abschnitt der Maske außerdem Haltepunkte (12) aufweist, die aus mindestens einem Schuss- oder Kettfaden hergestellt sind, der über dem elastischen Faden (6) schwebt, um ihn am Gewebeabschnitt (1) zu halten.

6. Maske nach einem der vorhergehenden Ansprüche, wobei die metallischen und elastischen Garne als Kettgarne in das Gewebe eingewebt sind.

7. Maske nach einem der vorhergehenden Ansprüche, wobei die Umfangsseiten (4) der Maske mit Schmelzgarnen versehen sind.

8. Verfahren zur Herstellung einer Maske nach einem der vorhergehenden Ansprüche, umfassend den Schritt des Bereitstellens einer Vielzahl von Stoffgarnen, um einen gewebten Stoff herzustellen; **dadurch gekennzeichnet, dass** es den Schritt des Webens von Kett- und Schussgarnen umfasst, wobei die Kett- oder Schussgarne mindestens eines aus einem plastisch biegbaren Garn und mindestens einem Garn mit einem elastischen Faden (6) umfassen, um mindestens einen Streifen (2) aus plastisch biegbarem Material für einen Nasenclip und ein Halteband (3) zum Halten des Maskenkörpers am Gesicht des Benutzers bereitzustellen.

9. Verfahren nach Anspruch 8, wobei das Gewebe auf dem Webstuhl eine Vielzahl von Gesichtsmasken umfasst, die schließlich geschnitten werden, nachdem das Gewebe vom Webstuhl entfernt wurde und Endbearbeitungsschritte durchlaufen hat.

10. Verfahren nach Anspruch 8 oder 9, wobei Schmelzgarne mit den Stoffgarnen verwoben werden, um Schmelzregionen (4) entlang der Seiten des Maskengewebes (1) bereitzustellen.

11. Verfahren nach Anspruch 10, das außerdem einen Wärmebehandlungsschritt umfasst, wodurch niedrig schmelzende Schmelzgarne ausreichend weich werden oder schmelzen, um die Enden der Garne zusammenzuhalten.

12. Verfahren nach einem der Ansprüche 8 bis 11, das außerdem Endbearbeitungsschritte umfasst, einschließlich mindestens eines von Oberflächenbehandlung, Beschneiden, Falten oder Kleben.

13. Verfahren nach einem der Ansprüche 8 bis 12, wobei die Maske außerdem mehrere lange Flottierungen der elastischen Fäden auf einer Seite der Maske und mehrere Haltepunkte (12) umfasst, die durch mindestens einen Schuss- oder Kettfaden gebildet werden, der über dem elastischen Faden (6) schwebt, um ihn am Gewebeabschnitt (1) zu halten, wobei das Verfahren den Schritt des Brechens der Haltegarne (12) umfasst, um eine lange Schlaufe des elastischen Fadens (6) bereitzustellen.

## Revendications

1. Masque respiratoire comprenant un corps de tissu de masque (1), une bande de matériau plastiquement pliable (2) et des sangles de maintien (3, 6) pour maintenir le corps de masque sur le visage d'un utilisateur, le corps de masque étant fait d'un tissu textile **caractérisé en ce que** : ledit tissu textile a des fils de trame et des fils de chaîne, ladite bande de matériau plastiquement pliable comprend au moins un fil tissé avec ledit tissu de corps de masque et/ou lesdites sangles de maintien comprennent au moins un fil tissé avec ledit tissu de corps de masque.

2. Masque selon la revendication 1, dans lequel ladite bande de matériau pliable est constituée d'au moins un, et de préférence d'une pluralité de fils de filaments métalliques (m), de préférence enduits d'une résine.

3. Masque selon la revendication 1 ou 2, dans lequel lesdites sangles de maintien (3) sont constituées d'au moins un filet élastique (6) tissé dans le corps du tissu au cours de l'étape de tissage.

4. Masque selon la revendication 3, dans lequel ledit filet élastique est étroitement tissé dans la région du tissu qui est proche des côtés de masque (8, 9), ledit filet (6) ayant au moins un long flotteur sur les fils de chaîne (ou de trame), de préférence plusieurs longs flotteurs dans une région centrale du corps de tissu de masque (1).

5. Masque selon la revendication 4, dans lequel ladite partie centrale du masque comprend en outre des points de maintien (12) constitués d'au moins un fil de trame ou de chaîne flottant sur ledit filet élastique (6) pour le maintenir sur la partie de tissu (1).

6. Masque selon l'une quelconque des revendications précédentes, dans lequel lesdits fils métalliques et élastiques sont tissés dans le tissu en tant que fils de chaîne.

7. Masque selon l'une quelconque des revendications précédentes, dans lequel les côtés périphériques (4) du masque sont pourvus de fils de fusion.

8. Procédé de fabrication d'un masque selon l'une quelconque des revendications précédentes, comprenant l'étape consistant à fournir une pluralité de fils de tissu pour produire un tissu textile ; **caractérisé en ce qu'**il comprend l'étape consistant à tisser des fils de chaîne et de trame, lesdits fils de chaîne ou de trame comprenant au moins un fil pliable plastiquement et au moins un fil de chaîne comprenant un filet élastique (6) pour fournir au moins une bande (2) de matériau pliable plastiquement pour un pince-nez et une sangle de maintien (3) pour maintenir le corps de masque sur le visage de l'utilisateur.

9. Procédé de la revendication 8, dans lequel le tissu sur le métier à tisser comprend une pluralité de masques qui sont finalement coupés après que le tissu a été retiré du métier à tisser et a subi des étapes de finition.

10. Procédé de la revendication 8 ou 9, dans lequel des fils de fusion sont tissés avec les fils de tissu pour fournir des zones de fusion (4) le long des côtés du tissu de masque (1).

11. Procédé de la revendication 10, comprenant en outre une étape de traitement thermique au cours de laquelle les fils de fusion à bas point de fusion se ramollissent ou fondent à un degré suffisant pour maintenir ensemble les extrémités des fils.

12. Procédé de l'une quelconque des revendications 8 à 11, comprenant en outre des étapes de finition comprenant au moins un des traitements de surface, le découpage, le pliage ou le collage.

13. Procédé de l'une quelconque des revendications 8 à 12, dans lequel ledit masque comprend en outre plusieurs longs flotteurs desdits filets élastiques sur un côté du masque et plusieurs points de maintien (12) réalisés par au moins un fil de trame ou de chaîne flottant sur ledit filet élastique (6) pour le maintenir sur la partie de tissu (1), le procédé comprenant l'étape consistant à rompre lesdits fils de maintien (12) pour fournir une longue boucle du filet élastique (6).
